# EUROPEAN PATENT APPLICATION

(11) **EP 3 563 781 A1**
(43) Date of publication of application: **06.11.2019**
(21) Application number: 17888871.5
(22) Date of filing: 13.12.2017
(51) Int. Cl.: A61B 17/29, A61B 34/37, B25J 15/08

(54) **FORCEPS ROBOT**

(30) Priority: 28.12.2016 JP 2016255037
(71) Applicant: Kawasaki Jukogyo Kabushiki Kaisha, Kobe-shi, Hyogo 650-8670 (JP)
(72) Inventor: KURIBAYASHI, Naoki, Kobe-shi, Hyogo 650-8670 (JP); WATANABE, Hideki, Kobe-shi, Hyogo 650-8670 (JP); ANADA, Tadashi, Kobe-shi, Hyogo 650-8670 (JP)
(74) Representative: Dehns
(86) International application number: PCT/JP2017/044785
(87) International publication number: WO 2018/123601

(57) **Abstract**

A robot forceps (1) includes an insertion tube (4) and a gripper (2) provided at a tip end of the insertion tube. The gripper includes: a first claw portion (21) and a second claw portion (22) arranged so as to be opposed to each other; and a first rotary actuator (30) connected to the first claw portion and configured to rotate the first claw portion by supply of an operating liquid into a first pressure chamber.

## Description

### Technical Field

The present invention relates to a robot forceps configured to open and close a gripper by utilizing liquid pressure.

### Background Art

Robot forceps have been conventionally used for endoscopic surgery and the like. For example, as shown in Fig. 7A, PTL 1 discloses a robot forceps 100 configured to open and close a gripper 103 by utilizing liquid pressure. The robot forceps 100 is manually operated.

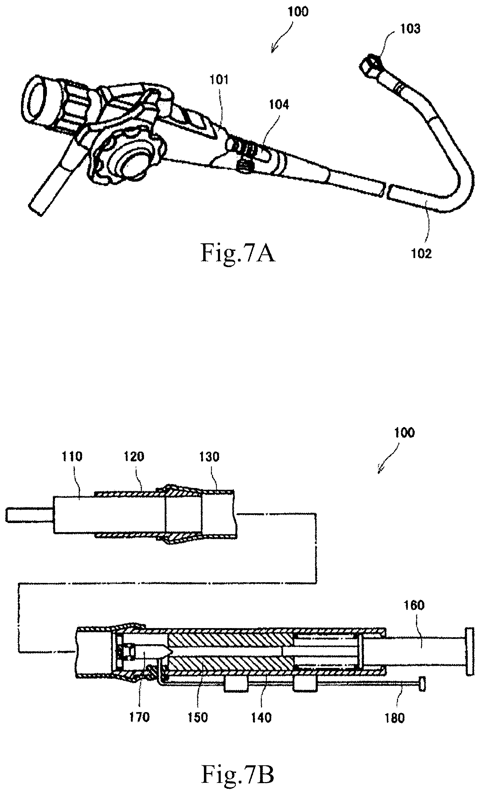

Specifically, the robot forceps 100 shown in Fig. 7A includes an operating portion main body 101, an insertion tube 102, and the gripper 103. An open/close operating portion 104 for the gripper 103 is provided at the operating portion main body 101.

As shown in Fig. 7B, the gripper 103 is coupled to a piston 110 configured to slide in a guide tube 120. To be specific, the piston 110 and the guide tube 120 are arranged inside a tip end portion of the insertion tube 102. Further, a tubular body 140 is provided at the open/close operating portion 104. The tubular body 140 and the guide tube 120 are connected to each other by a tube 130 extending in the insertion tube 102. A cylinder 150 is arranged inside the tubular body 140. A small-diameter portion of a pusher 160 is inserted into the cylinder 150, and a large-diameter portion of the pusher 160 is exposed from the tubular body 140.

When the pusher 160 is pushed into the tubular body 140, an operating liquid is pushed out from the cylinder 150, and this advances the piston 110 to open the gripper 103. When closing the gripper 103, a valve element 170 is set to an open state by operating a rod 180, and the pusher 160 is then pulled out from the tubular body 140. With this, the operating liquid is sucked into the cylinder 150, and this retracts the piston 110 to close the gripper 103.

### Citation List

### Patent Literature

PTL 1: Japanese Laid-Open Patent Application Publication No. 63-160631

### Summary of Invention

### Technical Problem

According to the robot forceps 100 shown in Figs. 7A and 7B, the gripper 103 is opened or closed by advancing or retracting the piston 110. Therefore, in order to open or close the gripper 103, in addition to the piston 110 configured to perform a linear motion, a mechanism configured to convert the linear motion into a rotational motion for opening or closing the gripper 103 is required. Therefore, the robot forceps 100 increases in size.

An object of the present invention is to provide a robot forceps which can be made smaller than conventional robot forceps.

### Solution to Problem

In order to solve the above problems, a robot forceps of the present invention includes: an insertion tube; and a gripper provided at a tip end of the insertion tube. The gripper includes: a first claw portion and a second claw portion arranged so as to be opposed to each other; and a first rotary actuator connected to the first claw portion and configured to rotate the first claw portion by supply of an operating liquid into a first pressure chamber.

According to the above configuration, since the first claw portion is rotated by the rotary actuator, an interval between the first claw portion and the second claw portion arranged so as to be opposed to each other can be changed, and therefore, the gripper can be opened or closed. Therefore, a mechanism configured to convert a motion into a rotational motion of the first claw portion when opening or closing the gripper is not required. On this account, the robot forceps of the present invention can be made smaller than conventional forceps.

The robot forceps may include a second rotary actuator connected to the second claw portion and configured to rotate the second claw portion by supply of the operating liquid into a second pressure chamber. According to this configuration, since the first claw portion and the second claw portion are respectively rotated by the first rotary actuator and the second rotary actuator, the gripper can be opened or closed. Further, since the first claw portion and the second claw portion rotate at the tip end of the insertion tube, an open/close position of the gripper can be changed not only to the front side of the insertion tube but also to each of both sides of the insertion tube in a rotational direction of the first and second claw portions.

The robot forceps may be configured such that: the first rotary actuator includes a first shaft portion, a first housing including a first depression having a fan shape, a first lid covering the first depression, and a first vane dividing a first space into a pair of first pressure chambers including the first pressure chamber, the first vane being configured to rotate about the first shaft portion by pressure of the operating liquid, the first space being formed by the first depression and the first lid; the second rotary actuator includes a second shaft portion, a second housing including a second depression having a fan shape, a second lid covering the second depression, and a second vane dividing a second space into a pair of second pressure chambers including the second pressure chamber, the second vane being configured to rotate about the second shaft portion by the pressure of the operating liquid, the second space being formed by the second depression and the second lid; and the first housing and the second housing are integrally formed. According to this configuration, since the first housing and the second housing are integrally formed, the size reduction and the cost reduction can be realized.

The robot forceps may further include a wrist portion interposed between the insertion tube and the gripper. The wrist portion may include a third rotary actuator configured to rotate the gripper relative to the insertion tube by supply of the operating liquid into a third pressure chamber. According to this configuration, since the gripper is rotated relative to the insertion tube by the third rotary actuator, the gripper can be bent (inclined) relative to the insertion tube.

The robot forceps may be configured such that: the third rotary actuator includes a third shaft portion, a third housing including a third depression having a fan shape, a third lid covering the third depression, and a third vane dividing a third space into a pair of third pressure chambers including the third pressure chamber, the third vane being configured to rotate about the third shaft portion by pressure of the operating liquid, the third space being formed by the third depression and the third lid; the third housing is fixed to the gripper; and the wrist portion includes a supporting portion fixed to the third shaft portion and connected to the insertion tube. According to this configuration, the third housing fixed to the gripper rotates relative to the supporting portion to which the third shaft portion is fixed. With this, the gripper can be bent (inclined) relative to the insertion tube connected to the supporting portion.

The robot forceps may be configured such that the third space is provided at a side of the third shaft portion which side is opposite to a side where the first claw portion and the second claw portion are provided. In addition, the robot forceps may further include: a pair of passage portions respectively connected to the pair of third pressure chambers formed by dividing the third space by the third vane, each of the passage portions being bent in a U shape; and a pair of pipes extending in the insertion tube and passing through between the supporting portion and the third rotary actuator, the pair of pipes being respectively connected to the pair of passage portions.

According to this configuration, a connection portion where the pipes and the passage portions are connected to one another can be arranged at a position close to the third shaft portion. With this, a movement distance of the pipe between the position of the pipe when the wrist portion is bent by the rotation of the third housing relative to the third shaft portion and the position of the pipe when the wrist portion is not bent can be made short. The projection of the pipe from the third rotary actuator when the wrist portion is bent can be made small.

### Advantageous Effects of Invention

The present invention has an effect of being able to provide a robot forceps which can be made smaller than conventional robot forceps.

### Brief Description of Drawings

Fig. 1 is a diagram of a robot forceps according to Embodiment 1 of the present invention.
Figs. 2A and 2B are perspective views each showing a tip end portion of the robot forceps of Fig. 1.
Fig. 3A is a diagram when viewing the robot forceps of Fig. 2A from a second-a side in a second direction. Fig. 3B is a diagram when viewing the robot forceps of Fig. 2B from the second-a side in the second direction.
Fig. 4A is a sectional view taken along line A-A of Fig. 3A showing the robot forceps. Fig. 4B is a diagram when viewing the robot forceps of Fig. 3A from a front side. Fig. 4C is a diagram when viewing the robot forceps of Fig. 3B from a rear side.
Fig. 5 is a diagram when viewing the robot forceps of Fig. 2A from a first-b side in a first direction.
Fig. 6A is a sectional view taken along line B-B of Fig. 5 showing the robot forceps. Fig. 6B is a sectional view taken along line C-C of Fig. 5 showing the robot forceps.
Fig. 7A is a perspective view showing a conventional robot forceps. Fig. 7B is a sectional view showing an internal structure of the conventional robot forceps.

### Description of Embodiments

Hereinafter, embodiments of the present invention will be specifically explained with reference to the drawings. In the following explanations and the drawings, the same reference signs are used for the same or corresponding components, and a repetition of the same explanation is avoided. For convenience of explanation, an axial direction of an insertion tube is referred to as a front-rear direction (a side where a gripper is provided is referred to as a front side, and a side where a drive unit is provided is referred to as a rear side). A direction perpendicular to the front-rear direction is referred to as a first direction (a side where a first claw portion is provided is referred to as a first-a side, and a side where a second claw portion is provided is referred to as a first-b side). A direction perpendicular to the front-rear direction and the first direction is referred to as a second direction (a side where a first coupling portion is provided is referred to as a second-a side, and a side where a second coupling portion is provided is referred to as a second-b side). However, directions of the robot forceps are not limited to these directions and are set arbitrarily.

### Embodiment 1

### Configuration of Robot Forceps

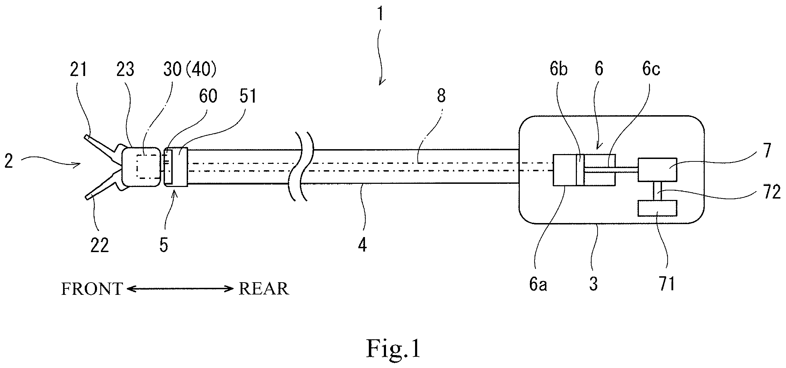

Fig. 1 shows a robot forceps 1 according to Embodiment 1 of the present invention. The robot forceps 1 opens or closes a gripper 2 by utilizing liquid pressure of an operating liquid. The operating liquid is not especially limited, and examples thereof include water, a physiological saline solution, and oil.

For example, the robot forceps 1 is used in a surgery assisting robot. In this case, the robot forceps 1 is attached to a manipulator of a slave-side apparatus, and a doctor performs remote manipulation of the robot forceps 1 by using a master-side apparatus. The manipulator freely changes the posture of the robot forceps 1 based on a fulcrum that is a hole provided on a skin of a patient.

Specifically, the robot forceps 1 includes a drive unit 3, an insertion tube 4, and the gripper 2. The insertion tube 4 extends from the drive unit 3 and is inserted into a body of the patient. The gripper 2 is provided at a tip end of the insertion tube 4 through a wrist portion 5.

The insertion tube 4 is a high-rigidity tube extending linearly. A pair of pipes 8 extends in the insertion tube 4. The pair of pipes 8 connect the drive unit 3 with rotary actuators 30, 40, and 60 of the gripper 2 and the wrist portion 5. Details of the gripper 2 and the wrist portion 5 will be described later.

The drive unit 3 includes supply/discharge mechanisms 6 configured to control the operating liquid. When moving each of a pair of claw portions 21 and 22 and the wrist portion 5 in one direction, the supply/discharge mechanism 6 supplies the operating liquid to the rotary actuator (30, 40, 60) through one of the pair of pipes 8. Further, when moving each of the pair of claw portions 21 and 22 and the wrist portion 5 in the other direction, the supply/discharge mechanism 6 supplies or discharges the operating liquid to or from the rotary actuator (30, 40, 60) through the other pipe 8. Each of the rotary actuators 30, 40, and 60 includes two pressure chambers, and the supply/discharge mechanisms 6 are connected to the respective pressure chambers through the pipes 8. Therefore, the robot forceps 1 includes six supply/discharge mechanisms 6.

Specifically, each of the supply/discharge mechanisms 6 includes a cylinder 6a and a piston 6b. The pipes 8 are connected to the cylinder 6a. The piston 6b is arranged inside the cylinder 6a. The cylinder 6a includes a tubular portion and a front wall. The tubular portion holds the piston 6b such that the piston 6b can slide. The front wall closes a front opening of the tubular portion. A pressure chamber is formed between the piston 6b and the front wall of the cylinder 6a and communicates with the pressure chambers of the rotary actuators 30, 40, and 60 through the pipes 8. The piston 6b is coupled to a linear motion mechanism 7 through a rod 6c. The linear motion mechanism 7 is coupled to an output shaft 72 of a motor 71 and converts a rotational motion of the output shaft 72 of the motor 71 into a linear motion of the rod 6c. The motor 71 is, for example, a servomotor.

### Configuration of Gripper

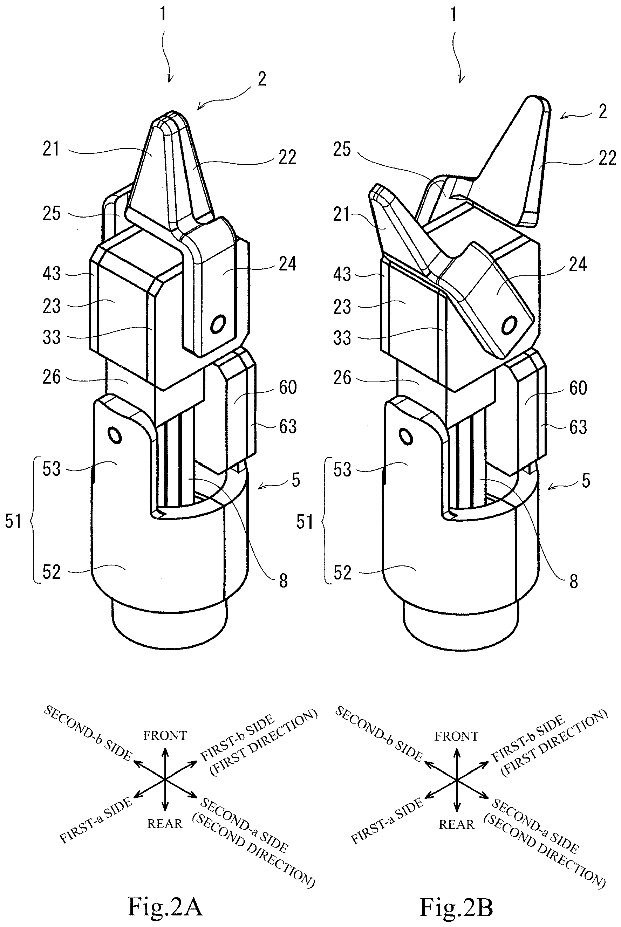

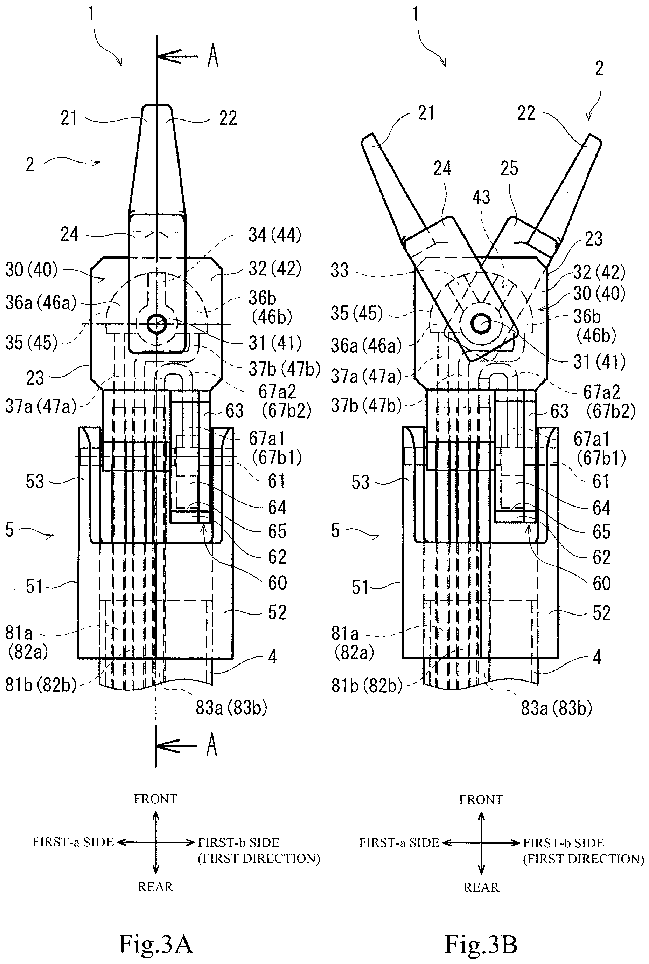

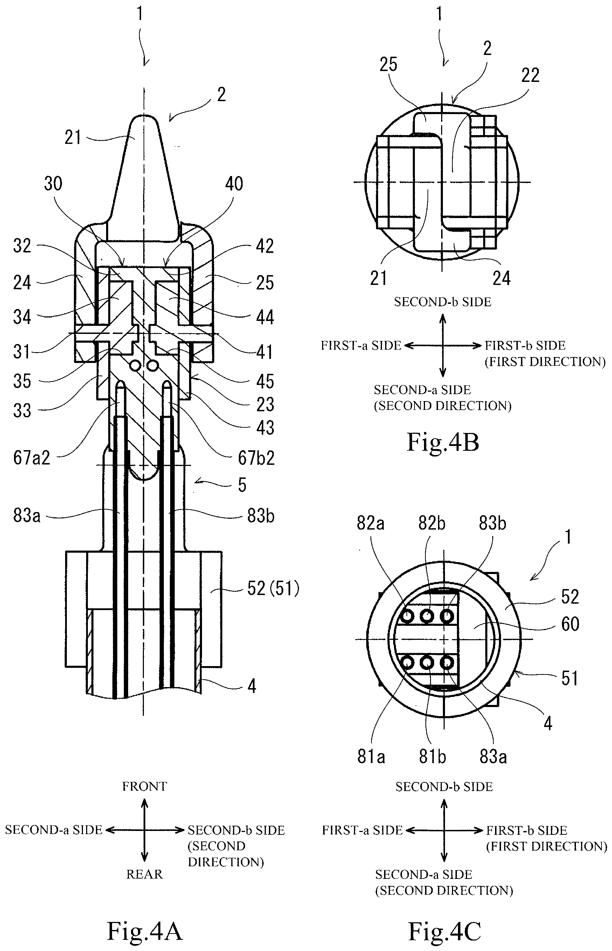

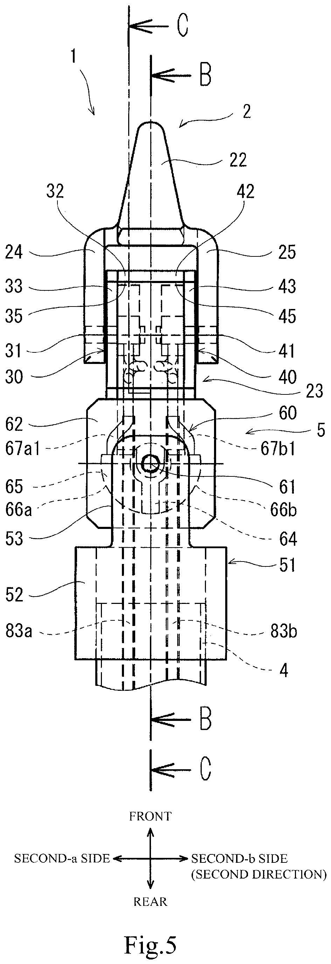

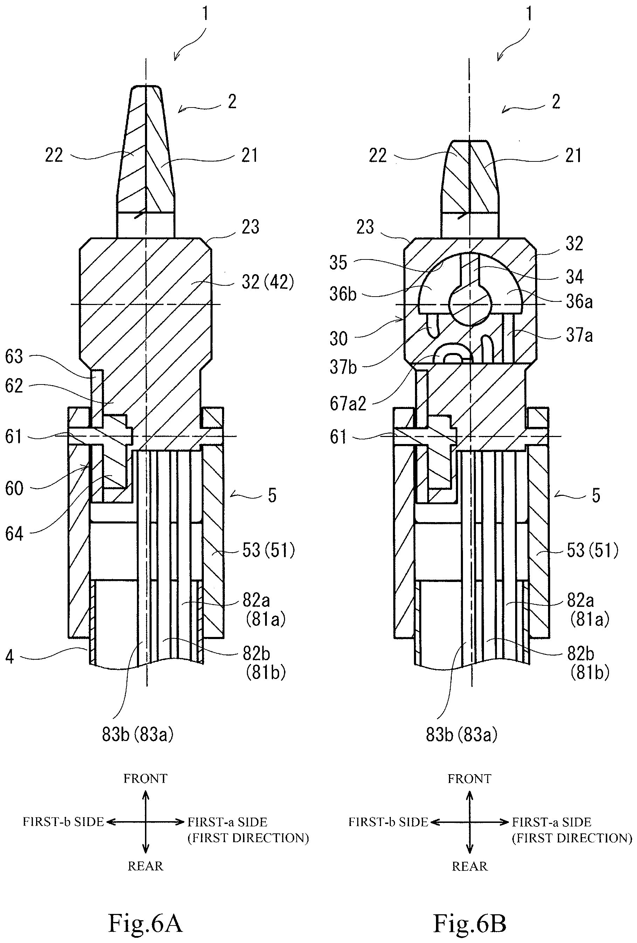

Figs. 2A to 6B show the gripper 2. The gripper 2 grips an affected part, a surgical needle, and the like. The gripper 2 includes a pair of claw portions (a first claw portion 21 and a second claw portion 22) and a first supporting portion 23. The first claw portion 21 and the second claw portion 22 are arranged so as to be opposed to each other. The first claw portion 21 and the second claw portion 22 move relative to each other so as to be able to change an interval therebetween.

For example, the first claw portion 21 is a triangular flat plate in a plan view. A vertex of the triangular flat plate is located at the front side, and a base of the triangular flat plate is connected to the first supporting portion 23 by a first coupling portion 24 so as to be spaced apart from the first supporting portion 23. The first coupling portion 24 extends from a bottom portion of the first claw portion 21 toward the second-a side in the second direction, is bent toward the rear side, and extends along a second-direction second-a side surface of the first supporting portion 23. A rear portion of the first coupling portion 24 is connected to a first shaft portion 31 of a first rotary actuator 30.

For example, the second claw portion 22 is a triangular flat plate in a plan view. A vertex of the triangular flat plate is located at the front side, and a base of the triangular flat plate is connected to the first supporting portion 23 by a second coupling portion 25 so as to be spaced apart from the first supporting portion 23. The second coupling portion 25 extends from a bottom portion of the second claw portion 22 toward the second-b side in the second direction, is bent toward the rear side, and extends along a second-direction second-b side surface of the first supporting portion 23. A rear portion of the second coupling portion 25 is connected to a second shaft portion 41 of a second rotary actuator 40.

The first supporting portion 23 includes a main body having a substantially rectangular solid shape. The first rotary actuator 30 and the second rotary actuator 40 are provided at the first supporting portion 23.

The first rotary actuator 30 is connected to the first claw portion 21 and rotates the first claw portion 21. The first rotary actuator 30 includes the first shaft portion 31, a first housing 32, a first lid 33, and a first vane 34. The first housing 32 is provided at the main body of the first supporting portion 23 and is arranged at, for example, a second-direction second-a side in the main body of the first supporting portion 23. A first depression 35 is provided at the first housing 32 and includes a bottom surface having a fan shape (for example, a semi-circular shape). The fan-shaped circular-arc portion of the first depression 35 is located in front of a linear portion of the first depression 35, and the fan shape of the first depression 35 projects toward the gripper 2 located at the front side. An opening of the first depression 35 is covered with the first lid 33, and with this, a first internal space is provided at the first housing 32. The first internal space is located closer to the first and second claw portions 21 and 31 than the first shaft portion 31. The first internal space is filled with the operating liquid.

The first shaft portion 31 is provided at a center of the fan shape of the first depression 35. The first shaft portion 31 extends in the second direction and penetrates the first lid 33. The first shaft portion 31 is connected to the first coupling portion 24 of the gripper 2. In the first internal space, the first vane 34 is connected to the first shaft portion 31. The first vane 34 is a plate-shaped body and extends from the first shaft portion 31 in a radial direction of the fan shape of the first depression 35. The first vane 34 divides the first internal space into a pair of first pressure chambers (a first-a pressure chamber 36a and a first-b pressure chamber 36b).

A connecting port of the first-a pressure chamber 36a is provided at the linear portion of the first depression 35 and is connected to a first-a passage portion 37a. The first-a passage portion 37a is provided at the main body of the first supporting portion 23. The first-a passage portion 37a extends in the front-rear direction and is connected to a first-a pipe 81a extending in the insertion tube 4. The first-a passage portion 37a and the first-a pipe 81a constitute a first-a pipe passage, and the first-a pressure chamber 36a is connected to the supply/discharge mechanism 6 (Fig. 1) through the first-a pipe passage.

A connecting port of the first-b pressure chamber 36b is provided at the linear portion of the first depression 35, and a first-b passage portion 37b is connected to the connecting port of the first-b pressure chamber 36b. The first-b passage portion 37b is provided at the main body of the first supporting portion 23. The first-b passage portion 37b extends in the front-rear direction and is connected to a first-b pipe 81b extending in the insertion tube 4. The first-b passage portion 37b and the first-b pipe 81b constitute a first-b pipe passage, and the first-b pressure chamber 36b is connected to the supply/discharge mechanism 6 (Fig. 1) through the first-b pipe passage.

The supply/discharge mechanisms 6 supply the operating liquid to the first-a and first-b pressure chambers 36a and 36b through the first-a and first-b pipe passages. The first vane 34 rotates about the first shaft portion 31 by a pressure difference between the operating liquid in the first-a pressure chamber 36a and the operating liquid in the first-b pressure chamber 36b. Then, the first claw portion 21 connected to the first vane 34 through the first shaft portion 31 moves.

The second rotary actuator 40 is connected to the second claw portion 22 and rotates the second claw portion 22. The second rotary actuator 40 includes the second shaft portion 41, a second housing 42, a second lid 43, and a second vane 44. The second internal space is formed by a second depression 45 of the second housing 42 and the second lid 43 and is divided into a pair of second pressure chambers (a second-a pressure chamber 46a and a second-b pressure chamber 46b) by the second vane 44. A second-a passage portion 47a connected to the second-a pressure chamber 46a and a second-a pipe 82a connected to the second-a passage portion 47a constitute a second-a pipe passage, and the second-a pressure chamber 46a is connected to the supply/discharge mechanism 6 (Fig. 1) through the second-a pipe passage. A second-b passage portion 47b connected to the second-b pressure chamber 46b and a second-b pipe 82b connected to the second-b passage portion 47b constitute a second-b pipe passage, and the second-b pressure chamber 46b is connected to the supply/discharge mechanism 6 (Fig. 1) through the second-b pipe passage.

The second rotary actuator 40 is arranged at the first supporting portion 23 so as to be symmetrical to the first rotary actuator 30 about a plane perpendicular to the second direction. Therefore, since the respective portions of the second rotary actuator 40 are the same as the respective portions of the first rotary actuator 30 except for the plane symmetry, explanations thereof are omitted.

As above, the first housing 32 and the second housing 42 which are arranged plane-symmetrically at the first supporting portion 23 are integrally formed at the main body of the first supporting portion 23. The first shaft portion 31 and the second shaft portion 41 are arranged on the same straight line.

### Configuration of Wrist Portion

Figs. 2A to 6B show the gripper 2 and the wrist portion 5. The wrist portion 5 is provided at the tip end of the insertion tube 4 and is connected to the gripper 2. The wrist portion 5 includes a second supporting portion 51 and a third rotary actuator 60. The second supporting portion 51 includes a cylindrical portion 52 and a pair of extending portions 53, and the cylindrical portion 52 is attached to the tip end of the insertion tube 4.

Each of the extending portions 53 is a substantially plate-shaped body and extends from the cylindrical portion 52 toward the front side. The pair of extending portions 53 are arranged so as to be opposed to each other in the first direction, and cutout portions are provided between the pair of extending portions 53 in a circumferential direction of the cylindrical portion 52.

The third rotary actuator 60 is fixed to the first supporting portion 23 of the gripper 2 and rotates the gripper 2 relative to the insertion tube 4. The third rotary actuator 60 includes a third shaft portion 61, a third housing 62, a third lid 63, and a third vane 64. The third housing 62 has, for example, a rectangular solid shape. A front surface of the third housing 62 is connected to a rear surface of the first supporting portion 23. The third housing 62 is provided between the pair of extending portions 53 so as to be located at the first-direction first-b side of a center between the pair of extending portions 53. A third depression 65 is provided at the third housing 62 and includes a bottom surface having a fan shape (for example, a semi-circular shape). The fan-shaped circular-arc portion of the third depression 65 is located at the rear side of a linear portion of the third depression 65, and the fan shape of the first depression 35 projects toward the insertion tube 4 located at the rear side. An opening of the third depression 65 is covered with the third lid 63, and with this, a third internal space is provided at the third housing 62. The third internal space is located closer to a side (a side where the insertion tube 4 is provided) than the third shaft portion 61, the side being opposite to a side where the first and second claw portions 21 and 31 are provided. The third internal space is filled with the operating liquid.

The third shaft portion 61 is provided at a center of the fan shape of the third depression 65. The third shaft portion 61 extends in the first direction and penetrates the third housing 62 and the third lid 63. The third shaft portion 61 is connected to the pair of extending portions 53. The third vane 64 is a plate-shaped body, and one end thereof is connected to the third shaft portion 61. The third vane 64 extends from the third shaft portion 61 in a radial direction of the fan shape of the third depression 65. The third vane 64 divides the third internal space into a pair of third pressure chambers (a third-a pressure chamber 66a and a third-b pressure chamber 66b).

A connecting port of the third-a pressure chamber 66a is provided at the linear portion of the third depression 65, and a third-a1 passage portion (passage portion) 67a1 is connected to the connecting port of the third-a pressure chamber 66a. The third-a1 passage portion 67a1 is provided at the third housing 62. The third-a1 passage portion 67a1 extends toward the front side and is connected to a third-a2 passage portion (passage portion) 67a2. The third-a2 passage portion 67a2 is provided at the main body of the first supporting portion 23. The third-a2 passage portion 67a2 extends from a rear end surface of the main body of the first supporting portion 23 toward the front side and is bent in a U shape. The third-a2 passage portion 67a2 then extends toward the rear side and is connected to a third-a pipe (pipe) 83a extending in the insertion tube 4. The third-a1 passage portion 67a1, the third-a2 passage portion 67a2, and the third-a pipe 83a constitute a third-a pipe passage, and the third-a pressure chamber 66a is connected to the supply/discharge mechanism 6 (Fig. 1) through the third-a pipe passage.

A connecting port of the third-b pressure chamber 66b is provided at the linear portion of the third depression 65, and a third-b 1 passage portion (passage portion) 67b1 is connected to the connecting port of the third-b pressure chamber 66b. The third-b1 passage portion 67b1 is provided at the third housing 62. The third-b 1 passage portion 67b1 extends toward the front side and is connected to a third-b2 passage portion (passage portion) 67b2. The third-b2 passage portion 67b2 is provided at the main body of the first supporting portion 23. The third-b2 passage portion 67b2 extends from the rear end surface of the main body of the first supporting portion 23 and is bent in a U shape. The third-b2 passage portion 67b2 then extends toward the rear side and is connected to a third-b pipe (pipe) 83b extending in the insertion tube 4. The third-b1 passage portion 67b1, the third-b2 passage portion 67b2, and the third-b pipe 83b constitute a third-b pipe passage. The third-b pressure chamber 66b is connected to the supply/discharge mechanism 6 (Fig. 1) through the third-b pipe passage.

The third rotary actuator 60 is arranged at one side (second-b side) in the second supporting portion 51, and a connection portion 26 where the passage portions 37a, 37b, 47a, 47b, 67a2, and 67b2 of the pipe passages and the pipes 8 are connected to one another is arranged at the other side (second-a side) in the second supporting portion 51. The pipes 8 include the first-a pipe 81a, the first-b pipe 81b, the second-a pipe 82a, the second-b pipe 82b, the third-a pipe 83a, and the third-b pipe 83b. Hereinafter, these pipes 81a, 81b, 82a, 82b, 83a, and 83b may be collectively called the pipes 8 when it is unnecessary to distinguish these pipes 81a, 81b, 82a, 82b, 83a, and 83b. The first-a pipe 81a and the first-b pipe 81b form a pair. The second-a pipe 82a and the second-b pipe 82b form a pair. The third-a pipe 83a and the third-b pipe 83b form a pair.

As above, the third rotary actuator 60 is arranged between the first-b side extending portion 53 of the second supporting portion 51 and a group of the pipes 8 and the connection portion 26. The pipes 8 and the connection portion 26 are arranged between the first-a side extending portion 53 of the second supporting portion 51 and the third rotary actuator 60. The pipes 8 are parallel to each other and extend in the insertion tube 4. Among these pipes 8, a pair of pipes 8 lined up in the second direction sandwich the third shaft portion 61 of the third rotary actuator 60 and are arranged so as to be symmetrical to each other about a center line extending in the second direction in the second supporting portion 51 and the insertion tube 4.

### Method of Operating Robot Forceps

As shown in Figs. 1 to 6B, the supply/discharge mechanisms 6 supply or discharge the operating liquid to or from the first and second rotary actuators 30 and 40 of the gripper 2 through the pipe passages. The operating liquid flows through the connecting ports by the pipe passages to flow into the pressure chambers 36a, 36b, 46a, and 46b of the first and second rotary actuators 30 and 40 or flow out from the pressure chamber 36a, 36b, 46a, and 46b of the first and second rotary actuators 30 and 40. The pressure of the operating liquid is applied to the first vane 34 located between the adjacent pressure chambers 36a and 36b, and the first vane 34 and the first shaft portion 31 connected to the first vane 34 rotate by the pressure difference between the adjacent pressure chambers 36a and 36b. The pressure of the operating liquid is applied to the second vane 44 located between the adjacent pressure chambers 46a and 46b, and the second vane 44 and the second shaft portion 41 connected to the second vane 44 rotate by the pressure difference between the adjacent pressure chambers 46a and 46b. In accordance with these rotations, the first claw portion 21 connected to the first shaft portion 31 by the first coupling portion 24 is inclined relative to the first supporting portion 23 at which the first rotary actuator 30 is provided, and the second claw portion 22 connected to the second shaft portion 41 by the second coupling portion 25 is inclined relative to the first supporting portion 23 at which the second rotary actuator 40 is provided.

For example, the first internal space constituted by the adjacent pressure chambers 36a and 36b has a semi-circular shape, and the second internal space constituted by the adjacent pressure chambers 46a and 46b has a semi-circular shape. In this case, each of the first and second vanes 34 and 44 rotates about 180° in the first direction (about ±90° with respect to the front-rear direction), and each of the first and second claw portions 21 and 22 respectively connected to the first and second vanes 34 and 44 inclines about 180° in the first direction. It should be noted that "about 180°" denotes a value including 180°, a manufacturing error, and the like, and "about 90°" denotes a value including 90°, a manufacturing error, and the like.

The first claw portion 21 and the second claw portion 22 which inline as above move relative to each other, and therefore, an interval between the first claw portion 21 and the second claw portion 22 arranged so as to be opposed to each other can be widened or narrowed. By narrowing the interval between the first claw portion 21 and the second claw portion 22 (i.e., by closing the gripper 2), the first claw portion 21 and the second claw portion 22 can sandwich and grip a grip object, such as tissue or an instrument. On the other hand, by widening the interval between the first claw portion 21 and the second claw portion 22 (i.e., by opening the gripper 2), the first claw portion 21 and the second claw portion 22 can release the grip object.

The first claw portion 21 is driven by the first rotary actuator 30, and the second claw portion 22 is driven by the second rotary actuator 40. Therefore, the first claw portion 21 and the second claw portion 22 can move independently and can also move in conjunction with each other. Further, one of the first claw portion 21 and the second claw portion 22 may be fixed, and only the other of the first claw portion 21 and the second claw portion 22 may move.

The supply/discharge mechanisms 6 supply or discharge the operating liquid to or from the third rotary actuator 60 of the wrist portion 5 through the pipe passages in the insertion tube 4. The operating liquid flows through the connecting ports by the pipe passages to flow into the pressure chambers 66a and 66b of the third rotary actuator 60 or flow out from the pressure chambers 66a and 66b of the third rotary actuator 60. The pressure of the operating liquid is applied to the third vane 64 located between the adjacent pressure chambers 66a and 66b, and the third housing 62 constituting the pressure chambers 66a and 66b rotates relative to the third vane 64 and the third shaft portion 61 connected to the third vane 64 by the pressure difference between the adjacent pressure chambers 66a and 66b. In accordance with this rotation, the first and second claw portions 21 and 22 connected to the third housing 62 through the first supporting portion 23 incline relative to a third supporting portion connected to the third shaft portion 61. A direction of this inclination is a direction (second direction) vertical to the third shaft portion 61 extending in the first direction. Regarding the inclination range, when the third internal space in which the third vane 64 is arranged has a semi-circular shape, the third housing 62 including the third internal space rotates about 180° (about ±90° with respect to the front-rear direction). Therefore, the first and second claw portions 21 and 22 connected to the third housing 62 also incline about 180°. It should be noted that "about 180°" denotes a value including 180°, a manufacturing error, and the like, and "about 90°" denotes a value including 90°, a manufacturing error, and the like.

### Actions and Effects

As above, the first rotary actuator 30 rotates the first claw portion 21, and the second rotary actuator 40 rotates the second claw portion 22. Therefore, a mechanism configured to convert a motion into a rotational motion used to drive the first and second claw portions 21 and 22 is not required. On this account, the robot forceps 1 can be made small, and therefore, the robot forceps 1 can be used in surgery at a limited space inside a body, for example.

The first vane 34 configured to drive the first claw portion 21 rotates in the first rotary actuator 30, and the second vane 44 configured to drive the second claw portion 22 rotates in the second rotary actuator 40. Therefore, it is unnecessary to additionally provide a space where the first and second second vanes 34 and 44 operate. Thus, the robot forceps 1 can be made small.

The first claw portion 21 is connected to the shaft portion 31 of the first rotary actuator 30 configured to drive the first claw portion 21, and the second claw portion 22 is connected to the shaft portion 41 of the second rotary actuator 40 configured to drive the second claw portion 22. Therefore, it is unnecessary to provide a power transmission mechanism, such as a wire and a pulley, between the first or second claw portion 21 or 22 and its driving portion. Therefore, power losses of the first and second claw portions 21 and 22 by friction and the like in the power transmission mechanism do not occur, and a decrease in grip force of the first and second claw portions 21 and 22 can be suppressed. Further, deterioration of the power transmission mechanism, such as abrasion of the wire by the pulley, does not occur, and therefore, a decrease in durability of the robot forceps 1 by the deterioration of the power transmission mechanism can be suppressed.

Further, the first and second claw portions 21 and 22 incline at about ±90° with respect to the front-rear direction by the first and second rotary actuators 30 and 40, and the movable ranges of the first and second claw portions 21 and 22 are wide. Further, the first claw portion 21 and the second claw portion 22 can rotate and move independently. Therefore, the first and second claw portions 21 and 22 can move not only toward the front side of the insertion tube 4 but also toward each of the first-a side and first-b side of the insertion tube 4 in the first direction (i.e., each of both sides of the insertion tube 4 in a rotational direction of the first and second claw portions 21 and 22), and the gripper 2 can be opened or closed. Therefore, it is unnecessary to provide a mechanism configured to move the first and second claw portions 21 and 22 toward the first-a side and the first-b side for changing an open/close position of the gripper 2 relative to the insertion tube 4. With this, the robot forceps 1 can be simplified. Further, it is unnecessary to control other mechanism, and therefore, the robot forceps 1 excels in operability.

The first housing 32 and the second housing 42 are integrally formed at the main body of the first supporting portion 23. Therefore, the cost and the production steps can be reduced as compared to a case where the first housing 32 and the second housing 42 are separately formed.

Each of the third-a2 and third-b2 passage portions 67a2 and 67b2 is bent in a U shape in the first direction, and the connection portion 26 where the third-a2 and third-b2 passage portions 67a2 and 67b2 are respectively connected to the third-a and third-b pipes 83a and 83b is arranged between the third rotary actuator 60 and the first-a side extending portion 53 of the second supporting portion 51. Therefore, the projections of the third-a and third-b pipes 83a and 83b when the wrist portion 5 inclines and is bent can be made small, and the differences of the lengths of the third-a and third-b pipes 83a and 83b can be made small.

To be specific, if the connecting ports of the third rotary actuator 60 are provided at the rear portions of the third-a and third-b pressure chambers 66a and 66b, the passage portions connected to these connecting ports extend toward the rear side and are connected to the third-a and third-b pipes 83a and 83b at the rear portion of the third housing 62. Therefore, the connection portion 26 where the passage portions and the third-a and third-b pipes 83a and 83b are connected to one another is located at the rear portion of the third rotary actuator 60 and provided away from the third shaft portion 61. On this account, when the wrist portion 5 is bent by the rotation of the third rotary actuator 60, the third-a and third-b pipes 83a and 83b extend from the third rotary actuator 60 in the second direction and are then bent toward the rear side. Therefore, the third-a and third-b pipes 83a and 83b project from the third rotary actuator 60. Further, the lengths of the third-a and third-b pipes 83a and 83b when the wrist portion 5 is bent and the lengths of the third-a and third-b pipes 83a and 83b when the wrist portion 5 is not bent are largely different from each other.

On the other hand, the connecting ports of the pressure chambers 66a and 66b of the third rotary actuator 60 are provided at the front side, and the third-a2 and third-b2 passage portions 67a2 and 67b2 connected to the connecting ports through the third-a 1 and third-b 1 passage portions 67b1 are bent in a U shape and connected to the third-a and third-b pipes 83a and 83b, respectively. With this, the connection portion 26 where the third-a2 and third-b2 passage portions 67a2 and 67b2 and the third-a and third-b pipes 83a and 83b are connected to one another can be provided close to the third shaft portion 61. Therefore, the lengths of the third-a and third-b pipes 83a and 83b when the wrist portion 5 is bent and the lengths of the third-a and third-b pipes 83a and 83b when the wrist portion 5 is not bent are not so different from each other. On this account, unstretchable pipes can be used as the third-a and third-b pipes 83a and 83b.

When the wrist portion 5 is bent, the third-a and third-b pipes 83a and 83b extend from the first supporting portion 23 in the second direction and are then bent toward the rear side. The connection portion 26 is located between the third rotary actuator 60 and the first-a side extending portion 53 of the second supporting portion 51 and at the rear portion of the first supporting portion 23. Therefore, the third-a and third-b pipes 38a and 83b can be prevented from projecting from the third rotary actuator 60.

The third rotary actuator 60 is arranged at the first-b side in the second supporting portion 51, and the connection portion 26 where the passage portions of the pipe passages and the pipes 8 are connected to one another is arranged at the first-a side in the second supporting portion 51. With this, since the connection portion 26 is not provided at the first-b side of the third rotary actuator 60, the third lid 63 is arranged at the first-b side of the third rotary actuator 60. Therefore, internal machining, such as the third depression 65 of the third housing 62, can be performed from the first-b side. Further, the third lid 63 can be easily attached to the third housing 62 from the first-b side.

### Other Embodiments

The present invention is not limited to the above embodiment, and various modifications may be made within the scope of the present invention.

For example, in the above embodiment, at least one of an inner surface of the first depression 35 of the first rotary actuator 30 and an end surface of the first vane 34 contacting the inner surface of the first depression 35 may be subjected to a surface treatment, such as coating. With this, liquid tightness between the first depression 35 and the first vane 34 can be secured, and movement accuracy of the first claw portion 21 coupled to the first vane 34 can be kept high. In addition, the abrasion of these surfaces can be reduced, and the durability of these surfaces can be improved. Similarly, at least one of an inner surface of the second depression 45 of the second rotary actuator 40 and an end surface of the second vane 44 contacting the inner surface of the second depression 45 may be subjected to a surface treatment, such as coating, and at least one of an inner surface of the third depression 65 of the third rotary actuator 60 and an end surface of the third vane 64 contacting the inner surface of the third depression 65 may be subjected to a surface treatment, such as coating.

In the above embodiment, both the first claw portion 21 and the second claw portion 22 are provided at the robot forceps 1 so as to be movable. However, the second claw portion 22 may be fixed. In this case, the second rotary actuator 40 connected to the second claw portion 22 may not be provided at the robot forceps 1. Even in this case, the first claw portion 21 connected to the first rotary actuator 30 moves relative to the second claw portion 22, so that the interval between the first claw portion 21 and the second claw portion 22 can be changed, and therefore, the gripper 2 can open or close.

In the above embodiment, the wrist portion 5 is constituted by the third rotary actuator 60. However, the wrist portion 5 may be constituted by a wire and a mechanism configured to operate the wire. In this case, by pulling the wire, the gripper 2 inclines and is bent relative to the insertion tube 4.

In the above embodiment, the linear motion mechanism 7 and the motor 71 may be omitted, and the piston 6b may be manually operated.

In the above embodiment, the insertion tube 4 may be flexible, and the pipes 8 may be guided by rings provided in the insertion tube 4 at predetermined intervals.

In the above embodiment, the supply/discharge mechanism 6 does not necessarily have to include the cylinder 6a and the piston 6b and may be a small rotary pump. In this case, a gear box may be interposed between the motor 71 and the pump.

### Reference Signs List

- 1: robot forceps
- 2: gripper
- 4: insertion tube
- 5: wrist portion
- 21: first claw portion
- 22: second claw portion
- 36a: first-a pressure chamber (first pressure chamber)
- 36b: first-b pressure chamber (first pressure chamber)
- 30: first rotary actuator
- 31: first shaft portion
- 32: first housing
- 33: first lid
- 34: first vane
- 35: first depression
- 40: second rotary actuator
- 41: second shaft portion
- 42: second housing
- 43: second lid
- 44: second vane
- 45: second depression
- 46a: second-a pressure chamber (second pressure chamber)
- 46b: second-b pressure chamber (second pressure chamber)
- 51: second supporting portion (supporting portion)
- 60: third rotary actuator
- 61: third shaft portion
- 62: third housing
- 63: third lid
- 64: third vane
- 65: third depression
- 66a: third-a pressure chamber (third pressure chamber)
- 66b: third-b pressure chamber (third pressure chamber)
- 67a1: third-a1 passage portion (passage portion)
- 67a2: third-a2 passage portion (passage portion)
- 67b1: third-b 1 passage portion (passage portion)
- 67b2: third-b2 passage portion (passage portion)
- 83a: third-a pipe (pipe)
- 83b: third-b pipe (pipe)

## Claims

1. A robot forceps comprising:
an insertion tube; and
a gripper provided at a tip end of the insertion tube, wherein
the gripper includes:
a first claw portion and a second claw portion arranged so as to be opposed to each other; and
a first rotary actuator connected to the first claw portion and configured to rotate the first claw portion by supply of an operating liquid into a first pressure chamber.

2. The robot forceps according to claim 1, further comprising a second rotary actuator connected to the second claw portion and configured to rotate the second claw portion by supply of the operating liquid into a second pressure chamber.

3. The robot forceps according to claim 2, wherein:
the first rotary actuator includes
a first shaft portion,
a first housing including a first depression having a fan shape,
a first lid covering the first depression, and
a first vane dividing a first space into a pair of first pressure chambers including the first pressure chamber, the first vane being configured to rotate about the first shaft portion by pressure of the operating liquid, the first space being formed by the first depression and the first lid;
the second rotary actuator includes
a second shaft portion,
a second housing including a second depression having a fan shape,
a second lid covering the second depression, and
a second vane dividing a second space into a pair of second pressure chambers including the second pressure chamber, the second vane being configured to rotate about the second shaft portion by the pressure of the operating liquid, the second space being formed by the second depression and the second lid; and
the first housing and the second housing are integrally formed.

4. The robot forceps according to any one of claims 1 to 3, further comprising a wrist portion interposed between the insertion tube and the gripper, wherein
the wrist portion includes a third rotary actuator configured to rotate the gripper relative to the insertion tube by supply of the operating liquid into a third pressure chamber.

5. The robot forceps according to claim 4, wherein:
the third rotary actuator includes
a third shaft portion,
a third housing including a third depression having a fan shape,
a third lid covering the third depression, and
a third vane dividing a third space into a pair of third pressure chambers including the third pressure chamber, the third vane being configured to rotate about the third shaft portion by pressure of the operating liquid, the third space being formed by the third depression and the third lid;
the third housing is fixed to the gripper; and
the wrist portion includes a supporting portion fixed to the third shaft portion and connected to the insertion tube.

6. The robot forceps according to claim 5, wherein the third space is provided at a side of the third shaft portion which side is opposite to a side where the first claw portion and the second claw portion are provided,
the robot forceps further comprising:
a pair of passage portions respectively connected to the pair of third pressure chambers formed by dividing the third space by the third vane, each of the passage portions being bent in a U shape; and
a pair of pipes extending in the insertion tube and passing through between the supporting portion and the third rotary actuator, the pair of pipes being respectively connected to the pair of passage portions.
